(19) Europäisches Patentamt
European Patent Office
Office européen des brevets

(11) **EP 4 270 213 A1**

(12) **EUROPEAN PATENT APPLICATION**
published in accordance with Art. 153(4) EPC

(43) Date of publication:
**01.11.2023 Bulletin 2023/44**

(21) Application number: **21820439.4**

(22) Date of filing: **21.01.2021**

(51) International Patent Classification (IPC):
*G06F 16/33* (2019.01)   *G06F 40/295* (2020.01)
*G06K 9/62* (2022.01)   *G16H 50/70* (2018.01)
*G16B 50/00* (2019.01)

(52) Cooperative Patent Classification (CPC):
**G06F 16/33; G06F 18/00; G06F 40/295;**
**G16B 50/00; G16H 50/70;** Y02A 90/10

(86) International application number:
**PCT/CN2021/073013**

(87) International publication number:
**WO 2022/134252 (30.06.2022 Gazette 2022/26)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **23.12.2020 CN 202011535972**

(71) Applicants:
• **BGI Genomics Co., Limited**
**Shenzhen, Guangdong 518000 (CN)**
• **Bgi Health (HK) Company Limited**
**Hong Kong 999077 (CN)**

(72) Inventors:
• **ZHOU, Jian**
**Shenzhen, Guangdong 518000 (CN)**
• **KONG, LingXiang**
**Shenzhen, Guangdong 518000 (CN)**
• **WANG, Jinan**
**Shenzhen, Guangdong 518000 (CN)**
• **HE, Zengquan**
**Shenzhen, Guangdong 518000 (CN)**

(74) Representative: **Hoffmann Eitle**
**Patent- und Rechtsanwälte PartmbB**
**Arabellastraße 30**
**81925 München (DE)**

(54) **METHOD FOR DETERMINING DEGREE OF ASSOCIATION WITH GENES, AND RELATED DEVICE**

(57)    The present application discloses a method and device for determining a degree of gene association. Through determining record data of target association record(s) of a disease description entry and a plurality of genes in each preset association database, inputting the record data into a preset entry-gene association matrix, and determining an association score of the disease description entry with respective to each of the plurality of genes in the preset association database, the degrees of association between the disease description entry and the plurality of genes can be rapidly obtained.

| | |
|---|---|
| determine a disease description entry in a case description text | S100 |

↓

| | |
|---|---|
| for each of a plurality of preset association databases, determine record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and input the record data into a preset entry-gene association matrix, to determine an association score of the disease description entry with respective to each of the plurality of genes in the preset association database | S200 |

↓

| | |
|---|---|
| determine degree of association between the disease description entry and each of the plurality of genes according to association scores of the disease description entry with respect to the gene identifiers corresponding to the plurality of genes in the plurality of preset association databases | S300 |

Fig. 1

**Description**

This application claims the priority of Chinese Application for Invention No. 202011535972.2, filed to the Patent Office of the People's Republic of China on Dec. 23, 2020, entitled "METHOD AND DEVICE FOR DETERMINING A DEGREE OF GENE ASSOCIATION", the entire content of which is incorporated herein by reference.

**FIELD OF THE INVENTION**

The application belongs to the technical field of gene association, and in particular to a method and device for determining a degree of gene association.

**BACKGROUND**

At present, with the deepening of medical research, the identification of pathogenic genes related to inherited diseases will provide an important auxiliary role for relevant personnel in the research and follow-up treatment of these inherited diseases.

An inherited disease is generally related to multiple pathogenic genes. A variety of clinical phenotype information described by disease description entries may exist in the case description text of patients with inherited diseases. Different clinical phenotypic information may be associated with one or more pathogenic genes. The pathogenic genes associated with different clinical phenotypic information may be the same or different. At present, 5181 kinds of inherited diseases and 15428 kinds of genes have been identified. How to quickly determine the association between disease description entries and genes in the patient's case description text has become an urgent technical problem for relevant personnel.

**DISCLOSURE OF THE INVENTION**

In view of the above problems, the present application provides a method and device for determining a degree of gene association to overcome the above problem or at least partially solve the above problem. The relevant technical solution is as follows.

A method for determining a degree of gene association, comprising:

determining a disease description entry in a case description text;
for a plurality of preset association databases:

determining record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes;
inputting the record data into a preset entry-gene association matrix, to determine an association score of the disease description entry with respective to each of the plurality of genes in the preset association database; and

determining degree of association between the disease description entry and each of the plurality of genes according to association scores of the disease description entry with respect to the gene identifiers corresponding to the plurality of genes in the plurality of preset association databases.

Optionally, the determining record data of target association record(s) in the preset association database according to the disease description entry and gene identifiers each corresponding to one of a plurality of genes comprises:

for any gene of the plurality of genes, determining record data of the target association record(s) of the disease description entry and a gene identifier corresponding to the gene in the preset association database respectively, wherein the record data of the target association record(s) comprises: a first number of association record(s) including both the gene identifier of the gene and the disease description entry, a second number of association record(s) including the disease description entry, a third number of association record(s) including the gene identifier of the gene, and a total number of association record(s) in the preset association database.

Optionally, the preset entry-gene association matrix is:

$$ M = \frac{T_1}{T_2} \times \log\left(\frac{T_s}{T_3 + 1}\right) $$

wherein, M is the association score of the disease description entry with respect to the gene identifier corresponding to the gene in the preset information gene database; $T_1$ is the first number, $T_2$ is the second number, $T_3$ is the third number; $T_S$ is the total number.

Optionally, the determining degree of association between the disease description entry and each of the plurality of genes according to association scores of the disease description entry with respect to the gene identifiers corresponding to the plurality of genes in the plurality of preset association databases comprises, for any gene:

weighting the association scores of the disease description entry with respect to the gene identifier corresponding to the gene in the plurality of preset information gene association databases to obtain an association value between the disease description entry and the gene; and
determining the degree of association between the disease description entry and the gene according to the association value.

Optionally, the determining the degree of association between the disease description entry and the gene according to the association value comprises:
determining the degree of association between the disease description entry and the gene according to

$$L = \frac{1}{\left(1 + e^{-z}\right)}$$

, wherein L is the degree of association between the disease description entry and the gene, e is the natural constant, and z is the association value.

Optionally, a training process of the preset entry-gene association matrix comprises:

inputting, as training information, record data of target association records including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively;
determining predicted degrees of association between the plurality of disease description entries and the plurality of genes respectively according to the determined predicted association scores;
determining errors between the predicted degrees of association and actual degrees of association of the plurality of disease description entries and the plurality of genes;
determining whether the errors are below a preset error threshold;
if yes, determining that the current entry-gene association training matrix is the entry-gene association matrix; and
if not, adjusting matrix parameters in the current entry-gene association training matrix through a gradient descent algorithm, using the adjusted entry-gene association training matrix as the current entry-gene association training matrix, and turning to the step of inputting, as training information, record data of target association record(s) including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively.

A device for determining a degree of gene association, comprising: a disease description entry determination unit, an association score determination unit, and an association degree determination unit;

wherein the disease description entry determination unit is used for determining a disease description entry in a case description text;
the association score determination unit is used for, for a plurality of preset association databases: determining record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and inputting the record data into a preset entry-gene association matrix, to determine an association score of the disease description entry with respective to each of the plurality of genes in the preset association database; and
the association degree determination unit is used for determining degree of association between the disease description entry and each of the plurality of genes according to association scores of the disease description entry with respect to the gene identifiers corresponding to the plurality of genes in the plurality of preset association databases.

Optionally, the association score determination unit is specifically used for, for any gene of the plurality of genes, respectively determining record data of target association record(s) including the disease description entry and a gene identifier corresponding to the gene in the preset association database, the record data of the target association record(s) comprising: a first number of association record(s) including both the gene identifier of the gene and the disease description entry, a second number of association record(s) including the disease description entry, a third number of association record(s) including the gene identifier of the gene, and a total number of association record(s) in the preset association database.

Optionally, the preset entry-gene association matrix is:

$$M = \frac{T_1}{T_2} \times \log\left(\frac{T_s}{T_3 + 1}\right)$$

wherein, M is the association score of the disease description entry with respect to the gene identifier corresponding to the gene in the preset information gene database; $T_1$ is the first number, $T_2$ is the second number, $T_3$ is the third number; $T_S$ is the total number.

Optionally, the association degree determination unit comprises an association value obtaining subunit and an association degree determination subunit,

wherein the association value obtaining subunit is used for, for any gene, weighting the association scores of the disease description entry with respect to the gene identifier corresponding to the gene in the plurality of preset information gene association databases to obtain an association value between the disease description entry and the gene;

the association degree determination subunit is used for determining the degree of association between the disease description entry and the gene according to the association value.

Optionally, the association degree determination subunit is used for determining the degree of association between the disease description entry and the gene according to

$$L = \frac{1}{\left(1 + e^{-z}\right)}$$

, wherein L is the degree of association between the disease description entry and the gene, e is the natural constant, and z is the association value.

Optionally, the device further comprises: a predicted association score determination unit, a predicted association degree determination unit, an error determination unit, an error comparison unit, an entry-gene association matrix determination unit, and a matrix parameter adjustment unit,

wherein the predicted association score determination unit is used for inputting, as training information, record data of target association records including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively;

the predicted association degree determination unit is used for determining predicted degrees of association between the plurality of disease description entries and the plurality of genes respectively according to the determined predicted association scores;

the error determination unit is used for determining errors between the predicted degrees of association and actual degrees of association of the plurality of disease description entries and the plurality of genes;

the error comparison unit is used for determining whether the errors are below a preset error threshold, if yes, triggering the entry-gene association matrix determination unit, or if not, triggering the matrix parameter adjustment unit;

the entry-gene association matrix determination unit is used for determining the current entry-gene association training matrix as the entry-gene association matrix;

the matrix parameter adjustment unit is used for adjusting matrix parameters in the current entry-gene association training matrix through a gradient descent algorithm, using the adjusted entry-gene association training matrix as the current entry-gene association training matrix, and triggering the predicted association score determination unit.

A storage medium on which a computer program is stored, which when executed by a processor implements the method for determining a degree of gene association according to any one described above.

A processor for executing a program, wherein the program when executed performs the method for determining a degree of gene association according to any one described above.

An electronic device comprising at least one processor, at least one memory coupled to the processor, and a bus; wherein, the processor communicates with the memory through the bus; the processor is used for invoking program instructions in the memory to perform the method for determining a degree of gene association according to any one described above.

A computer program product, when executed on an electronic device, is adapted to execute a program for initializing the steps of the method for determining a degree of gene association according to any one described above.

With the above technical scheme, the method and device for determining the degree of gene association provided in this application is capable of determining a disease description entry in a case description text. For a plurality of preset association databases, record data of target association record(s) in the preset association database is determined according to the disease description entry and gene identifiers each corresponding to one of a plurality of genes, wherein each of the preset association databases stores association records of the disease description entry and gene identifiers corresponding to at least one of the plurality of genes; the record data is inputted into a preset entry-gene association matrix, to determine an association score of the disease description entry with respective to each of the plurality of genes in the preset association database; and the degree of association between the disease description entry and each of the plurality of genes is determined according to association scores of the disease description entry with respect to the gene identifiers corresponding to the plurality of genes in the plurality of preset association databases. In this application, through determining record data of target association record(s) of a disease description entry and a plurality of genes in each preset association database, and inputting the record data into a preset entry-gene association matrix, an association score of the disease description entry with respective to each of the plurality of genes in the preset association database is determined, and the degrees of association between the disease description entry and the plurality of genes can be rapidly obtained.

The above is only an overview of the technical solution in this application. In order 'to understand the technical means of this application more clearly, it can be implemented in accordance with the content of this description, and in order to make the above and other objects, features, and advantages of the present application more comprehensible, specific embodiments of the present application will be given below.

## DESCRIPTION OF THE DRAWINGS

In order to more clearly explain the embodiments of the present application or the technical solutions in the prior art, a brief introduction will be given below for the drawings required to be used in the description of the embodiments or the prior art. It is obvious that, the drawings illustrated as follows are merely some of the embodiments of the present application. For a person skilled in the art, he or she may also acquire other drawings according to such drawings on the premise that no inventive effort is involved.

FIG. 1 shows a flow diagram of a method for determining a degree of gene association provided in some embodiments of the present application;
FIG. 2 shows a flow diagram of another method for determining a degree of gene association provided in some embodiments of the present application;
FIG. 3 shows a schematic diagram of a training process of a preset entry-gene association matrix provided in some embodiments of the present application;
FIG. 4 shows a flow diagram of still another method for determining a degree of gene association provided in some embodiments of the present application;
FIG. 5 shows a structure diagram of a device for determining a degree of gene association provided in some embodiments of the present application;
FIG. 6 shows a structure diagram of another device for determining a degree of gene association provided in some embodiments of the present application.

## DETAILED DESCRIPTION OF PREFERRED EMBODIMENTS

Exemplary embodiments of the present disclosure will now be described in detail with reference to the accompanying drawings. Although the exemplary embodiments of the present disclosure are shown in the drawings, it should be understood that the present disclosure can be implemented in various forms and should not be limited by the embodiments set forth herein. Rather, these embodiments are provided to enable a more thorough understanding of the present disclosure and to fully convey the scope of the present disclosure to those skilled in the art.

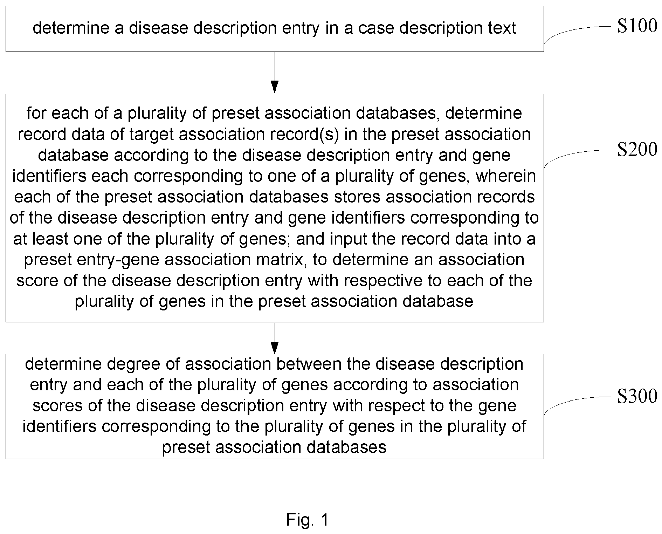

As shown in FIG. 1, the method for determining a degree of gene association provided in some embodiments of the present application may comprise:
S100: determining a disease description entry in a case description text.

The case description text can be a record of medical activities such as medical staff's examination and diagnosis of patients' diseases. Optionally, the case description text can be sentences or paragraphs composed of multiple words. The disease description entry can be an entry describing the clinical phenotype information of the patient's disease.

Optionally, the disease description entry can be HPO entry or inherited disease entry. HPO (human phenotype ontology) is a set of standard vocabulary describing abnormal phenotypes caused by human diseases. The standard vocabulary set includes multiple HPO entries. It is understandable that HPO entries can be Chinese words or words in other languages, such as English words. The inherited disease entry can be a professional name, and/or an abbreviation, and/or one of other user-defined names of diseases with genes as the main cause of the diseases. It should be noted that the user-defined names can be the colloquial expressions of the diseases in the industry. For example: the disease with the professional name of "bronchial asthma" can be abbreviated as "asthma", and it is often expressed in colloquialism with "wheezing". It is understandable that due to the continuous in-depth research on inherited diseases in the research field and the different actual needs in various application fields, industry personnel can create inherited disease entries by themselves according to research needs or actual application needs.

Optionally, in some embodiments of this application, the case description text can be segmented, and structured disease description entries can be extracted from the unstructured case description text through named entity recognition (NER) in natural language processing (NLP). The embodiments of this application can accurately extract disease description entries from the case description text by using the named entity recognition technology.

It can be understood that the embodiments of this application can also retrieve disease description entries from the case description text through preset disease description keywords.

It can be understood that the case description text can include one or more disease description entries, and the embodiments of this application can perform the method for determining the degree of gene association provided by the embodiments of this application on any disease description entry determined from the case description text.

S200: for a plurality of preset association databases, determining record data of one or more target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and inputting the record data into a preset entry-gene association matrix, to determine an association score of the disease description entry with respective to each of the plurality of genes in the preset association database.

The preset association database may be a database constructed based on a public disease database in the industry to associate mappable entries with gene identifiers. Optionally, public disease databases in the industry can include: OMIM (disease-related gene) database, ClinVar (genetic variation) database, HGMD (human gene mutation) database, HPO (standard terms of human phenotype) database and Orphanet (rare disease-related) database. The embodiments of this application can respectively build association databases for different public disease databases in the industry. Optionally, the preset association database can store association record(s) that map the association relationship between disease description entries and gene identifiers.

Optionally, the embodiments of this application can determine record data of target association record(s) in the preset association database according to a disease description entry and gene identifiers corresponding to a plurality of genes. Optionally, the record data of the target association record(s) can be the number of association record(s) including the disease description entry and any one of the plurality of genes in the preset association database, or the number of association record(s) including the gene identifier of any one of the plurality of genes in the preset association database.

Optionally, step S200 may include: for any gene of the plurality of genes, determining the record data of the target association record(s) of the disease description entry and a gene identifier corresponding to the gene in the preset association database respectively. The record data of the target association record(s) includes a first number of association record(s) including both the gene identifier of the gene and the disease description entry, a second number of association record(s) including the disease description entry, a third number of association record(s) including the gene identifier of the gene, and a total number of association record(s) in the preset association database.

It can be understood that the target association record(s) can be association record(s) in the preset association database, and may be association record(s) including both the gene identifier of the gene and the disease description entry in the preset association database, association record(s) including the disease description entry in the preset association database, or association record(s) including the gene identifier of the gene.

Optionally, the preset entry-gene association matrix may be:

$$M = \frac{T_1}{T_2} \times \log\left(\frac{T_s}{T_3 + 1}\right)$$

wherein, M is the association score of the disease description entry with respect to the gene identifier corresponding to the gene in the preset information gene database; $T_1$ is the first number, $T_2$ is the second number, $T_3$ is the third number, and $T_S$ is the total number.

In the embodiments of this application, through inputting the record data of the determined target association record(s) into the preset entry-gene association matrix, association scores outputted from the preset entry-gene association matrix can be obtained through calculation. It should be noted that the number of association scores of the determined disease description entry with respect to any one of the plurality of genes is the same as that of the plurality of preset association databases. For example, given that there are five preset association databases, for the disease description entry and any gene, in some embodiments of this application, association scores of the disease description entry with respect to the gene can be determined based on the five preset association databases using the preset entry-gene association matrix, that is, five association scores of the disease description entry with respect to the gene can be determined. It can be understood that the five association scores can be the same or different.

S300: determining degree of association between the disease description entry and each of the plurality of genes according to association scores of the disease description entry with respect to the gene identifiers corresponding to the plurality of genes in the plurality of preset association databases.

Optionally, in the embodiments of this application, for any gene, the association scores corresponding to the disease description entry and the gene identifier corresponding to the gene in the plurality of preset information-gene association databases are summed to obtain an association value of the disease description entry and the gene.

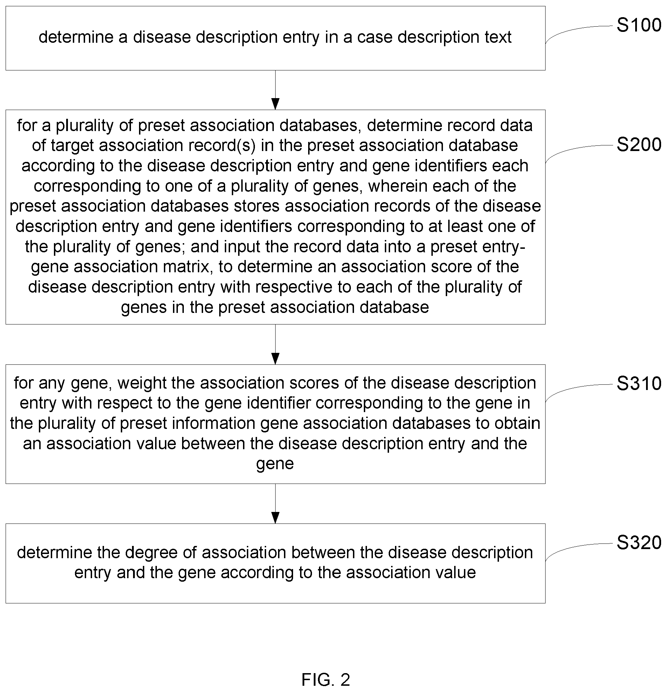

Optionally, based on the method shown in FIG.1, another method for determining a degree of gene association provided in some embodiments of the present application is shown in FIG. 2. Step S300 may comprise:

S310: for any gene, weighting the association scores of the disease description entry with respect to the gene identifier corresponding to the gene in the plurality of preset information gene association databases to obtain an association value between the disease description entry and the gene.

In some embodiments of this application, a weight can be set for each preset information-gene association database in advance, so that a more reliable association value between the disease description entry and the gene can be obtained after the weighting processing. For ease of understanding, it is illustrated here by an example. The weight of a preset information-gene association database A is set to w1, the weight of a preset information-gene association database B is set to w2, the weight of a preset information-gene association database C is set to w3, the weight of a preset information-gene association database D is set to w4, and the weight of a preset information-gene association database E is set to w5 in advance; the association score of the disease description entry with respect to a gene identifier of the gene is x1 corresponding to the preset information-gene association database A, x2 corresponding to preset information-gene association database B, x3 corresponding to preset information-gene association database C, x4 corresponding to preset information-gene association database D, and x5 corresponding to preset information-gene association database E. Through the weighting processing, the association value between the disease description entry and the gene is obtained as follows:

$$ z = x_1 w_1 + x_2 w_2 + x_3 w_3 + x_4 w_4 + x_5 w_5 $$

S320: determining the degree of association between the disease description entry and the gene according to the association value.

Optionally, step S320 may comprise: determining the degree of association between the disease description entry and the gene according to

$$ L = \frac{1}{\left(1 + e^{-z}\right)} $$

, wherein L is the degree of association between the disease description entry and the gene, e is the natural constant, and z is the association value.

This application provides a method for determining the degree of gene association, capable of determining a disease description entry in a case description text. For a plurality of preset association databases, record data of target association record(s) in the preset association database is determined according to the disease description entry and gene identifiers each corresponding to one of a plurality of genes, wherein each of the preset association databases stores association records of the disease description entry and gene identifiers corresponding to at least one of the plurality of genes; the record data is inputted into a preset entry-gene association matrix, to determine an association score of the disease description entry with respective to each of the plurality of genes in the preset association database; and the degree of association between the disease description entry and each of the plurality of genes is determined according to association scores of the disease description entry with respect to the gene identifiers corresponding to the plurality of genes in the plurality of preset association databases. In this application, through determining record data of target association records of a disease description entry and a plurality of genes in each preset association database, and inputting the record data into a preset entry-gene association matrix, an association score of the disease description entry with respective to each of the plurality of genes in the preset association database is determined, and the degrees of association between the disease description entry and the plurality of genes can be rapidly obtained.

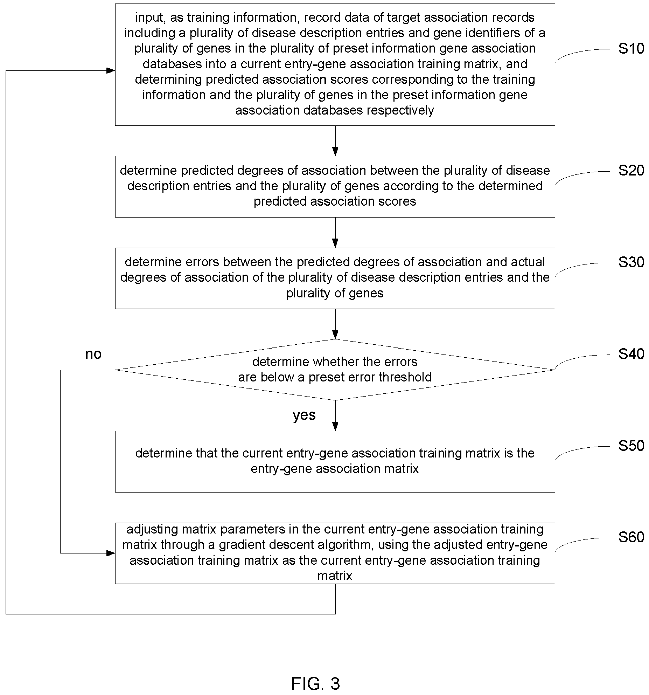

Optionally, as shown in FIG. 3, a process of training a preset entry-gene association matrix provided in some embodiments of this application may comprise: S10: inputting, as training information, record data of target association records including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively.

The embodiments of this application can initialize the entry-gene association training matrix at the beginning of training. Step S10 is similar to step S200. The embodiments of this application can successively input the record data of the target association records of a plurality of disease description entries and gene identifiers corresponding to a plurality of genes in a plurality of preset information-gene association databases into the current entry-gene association training matrix to obtain predicted association scores output from the current entry-gene association training matrix. Similarly, the determined number of predicted association scores of a disease description entry with respect to any one of the plurality of genes is the same as the number of the plurality of preset association databases.

S20: determining predicted degrees of association between the plurality of disease description entries and the plurality of genes according to the determined predicted association scores.

Optionally, in the embodiments of this application, for any gene, the association scores corresponding to the disease description entry and the gene identifier corresponding to the gene in the plurality of preset information gene association databases are summed to obtain an association value of the disease description entry and the gene.

Optionally, in the embodiments of this application, for any gene, the association scores corresponding to the disease description entry and the gene identifier corresponding to the gene in the plurality of preset information gene association databases are weighted to obtain an association value between the disease description entry and the gene.

In the embodiments of this application, the degree of association between the disease description entry and the gene can be determined according to the association value.

S30: determining errors between the predicted degrees of association and actual degrees of association of the plurality of disease description entries and the plurality of genes.

The actual degrees of association can be degrees of association between a plurality of disease description entries and a plurality of genes determined by professionals in the field. In the embodiments of this application, whether the training of current entry-gene association training matrix has been completed can be determined according to the errors between the actual degrees of association and the predicted degrees of association.

S40: determining whether the errors are below a preset error threshold; if yes, executing step S50, or if not, executing step S60.

The preset error threshold can be specified according to the actual needs in the embodiments of this application, which is not further limited herein.

S50: determining that the current entry-gene association training matrix is the entry-gene association matrix.

S60: adjusting matrix parameters in the current entry-gene association training matrix through a gradient descent algorithm, using the adjusted entry-gene association training matrix as the current entry-gene association training matrix, and turning to step S10.

The embodiments of this application adjusts the matrix parameters through a gradient descent algorithm, and can verify and train the entry-gene association training matrix again after adjusting the matrix parameters, so that more accurate association scores can be obtained using the finally obtained entry-gene association training matrix, further reducing errors between the actual degrees of association and the predicted degrees of association.

It can be understood that the embodiments of the invention can also adjust the weight of each preset information gene-association database through a gradient descent method when determining that the errors are not below the preset error threshold. By adjusting the weight of each preset information gene association database, the errors between the actual degrees of association and the predicted degrees of association can be reduced.

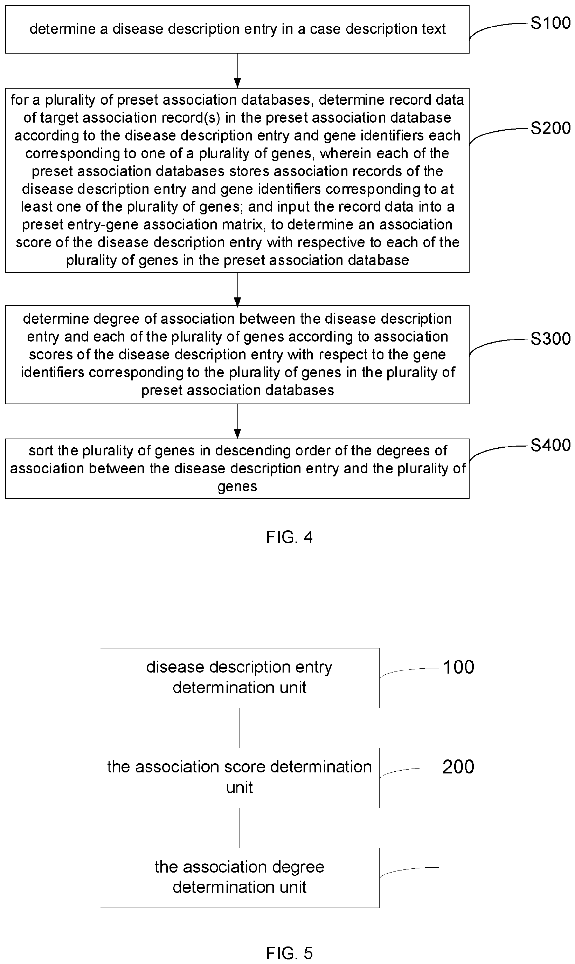

Optionally, based on the method shown in FIG.1, another method for determining the degree of gene association provided in some embodiments of the present application is shown in FIG. 4. After step S300, the method may further comprise:

S400: sorting the plurality of genes in descending order of the degrees of association between the disease description entry and the plurality of genes.

It can be understood that the higher the degree of association between the gene and the disease description entry, the more similar the disease clinical phenotype described by the disease description entry is to the phenotype of the gene. Sorting the plurality of genes after determining the degrees of association between the disease description entry and a plurality of genes is helpful for relevant technicians to identify and screen pathogenic genes of the disease corresponding to the disease description entry.

Corresponding to the above method embodiments, a device for determining a degree of gene association is further provided in some embodiments of this application. The structure of the device is shown in Fig. 5, and may comprise: a disease description entry determination unit 100, an association score determination unit 200, and an association degree determination unit 300.

The disease description entry determination unit 100 is used for determining a disease description entry in a case description text.

The case description text can be a record of medical activities such as medical staff's examination and diagnosis of patients' diseases. Optionally, the case description text can be sentences or paragraphs composed of multiple words. The disease description entry can be an entry describing the clinical phenotype information of the patient's disease.

Optionally, the disease description entry can be HPO entry or inherited disease entry. HPO (human phenotype ontology) is a set of standard vocabulary describing abnormal phenotypes caused by human diseases. The standard vocabulary set includes multiple HPO entries. It is understandable that HPO entries can be Chinese words or words in other languages, such as English words. The inherited disease entry can be a professional name, and/or an abbreviation, and/or one of other user-defined names of diseases with genes as the main cause of the diseases. It should be noted that the user-defined names can be the colloquial expressions of the diseases in the industry.

For example: the disease with the professional name of "bronchial asthma" can be abbreviated as "asthma", and it is often expressed in colloquialism with "wheezing". It is understandable that due to the continuous in-depth research on inherited diseases in the research field and the different actual needs in various application fields, industry personnel can create inherited disease entries by themselves according to research needs or actual application needs.

Optionally, the disease description entry determination unit 100 can segment the case description text, and then extract structured disease description entries from the unstructured case description text through named entity recognition (NER) in natural language processing (NLP). The embodiments of this application can accurately extract disease description entries from the case description text by using the named entity recognition technology.

It can be understood that the disease description entry determination unit 100 can also retrieve disease description entries from the case description text by presetting disease description keywords.

The association score determination unit 200 is used for, for a plurality of preset association databases: determining record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and inputting the record data into a preset entry-gene association matrix, to determine an association score of the disease description entry with respective to each of the plurality of genes in the preset association database.

The preset association database may be a database constructed based on a public disease database in the industry to associate mappable entries with gene identifiers. Optionally, public disease databases in the industry can include: OMIM (disease-related gene) database, ClinVar (genetic variation) database, HGMD (human gene mutation) database, HPO (standard terms of human phenotype) database and Orphanet (rare disease-related) database. Optionally, the preset association database can store association records that map the association relationship between disease description entries and gene identifiers.

Optionally, the association score determination unit 200 can determine record data of target association record(s) in the preset association database according to a disease description entry and gene identifiers corresponding to a plurality of genes. Optionally, the record data of the target association record(s) can be the number of association record(s) including the disease description entry and any one of the plurality of genes in the preset association database, or the number of association record(s) including the gene identifier of any one of the plurality of genes in the preset association database.

Optionally, the association score determination unit 200 is specifically used for, for any gene of the plurality of genes, determining the record data of the target association record(s) of the disease description entry and a gene identifier corresponding to the gene in the preset association database respectively. The record data of the target association record(s) includes: a first number of association record(s) including both the gene identifier of the gene and the disease description entry, a second number of association record(s) including the disease description entry, a third number of association record(s) including the gene identifier of the gene, and a total number of association record(s) in the preset association database.

It can be understood that the target association record(s) can be association record(s) in the preset association database, and may be association record(s) including both the gene identifier of the gene and the disease description entry in the preset association database, association record(s) including the disease description entry in the preset association database, or association record(s) including the gene identifier of the gene.

Optionally, the preset entry-gene association matrix may be:

$$M = \frac{T_1}{T_2} \times \log\left(\frac{T_s}{T_3 + 1}\right)$$

wherein, M is the association score of the disease description entry with respect to the gene identifier corresponding to the gene in the preset information gene database; $T_1$ is the first number, $T_2$ is the second number, $T_3$ is the third number, and $T_S$ is the total number.

The association score determination unit 200 is used for, through inputting the record data of the determined target association record(s) into the preset entry-gene association matrix, obtaining an association score outputted from the preset entry-gene association matrix through calculation. It should be noted that the number of association scores of the determined disease description entry with respect to any one of the plurality of genes is the same as that of the plurality of preset association databases.

The association degree determination unit 300 is used for determining degree of association between the disease description entry and each of the plurality of genes according to association scores of the disease description entry with respect to the gene identifiers corresponding to the plurality of genes in the plurality of preset association databases.

Optionally, the association degree determination unit 300 may be used for, for any gene: summing the association scores corresponding to the disease description entry and the gene identifier corresponding to the gene in the plurality of preset information gene association databases to obtain an association value of the disease description entry and the gene.

Optionally, the association degree determination unit 300 comprises an association value obtaining subunit and an association degree determination subunit.

The association value obtaining subunit is used for, for any gene, weighting the association scores of the disease description entry with respect to the gene identifier corresponding to the gene in the plurality of preset information gene association databases to obtain an association value between the disease description entry and the gene.

In the embodiments of this application, a weight can be set for each preset information-gene association database in advance, so that a more reliable association value between the disease description entry and the gene can be obtained after the weighting processing.

The association degree determination subunit is used for determining the degree of association between the disease description entry and the gene according to the association value.

Optionally, the association degree determination subunit is specifically used for determining the degree of association between the disease description entry and the gene according to

$$L = \frac{1}{\left(1 + e^{-z}\right)},$$

wherein L is the degree of association between the disease description entry and the gene, e is the natural constant, and z is the association value.

A device for determining the degree of gene association is provided in this application, capable of determining a disease description entry in a case description text. For a plurality of preset association databases, record data of target association record(s) in the preset association database is determined according to the disease description entry and gene identifiers each corresponding to one of a plurality of genes, wherein each of the preset association databases stores association records of the disease description entry and gene identifiers corresponding to at least one of the plurality of genes; the record data is inputted into a preset entry-gene association matrix, to determine an association score of the disease description entry with respective to each of the plurality of genes in the preset association database; and the degree of association between the disease description entry and each of the plurality of genes is determined according to association scores of the disease description entry with respect to the gene identifiers corresponding to the plurality of genes in the plurality of preset association databases. In this application, through determining record data of target association record(s) of a disease description entry and a plurality of genes in each preset association database, and inputting the record data into a preset entry-gene association matrix, an association score of the disease description entry with respective to each of the plurality of genes in the preset association database is determined, and the degrees of association between the disease description entry and the plurality of genes can be rapidly obtained.

Optionally, the device for determining a degree of gene association may further comprise: a predicted association score determination unit, a predicted association degree determination unit, an error determination unit, an error comparison unit, an entry-gene association matrix determination unit, and a matrix parameter adjustment unit.

The predicted association score determination unit is used for inputting, as training information, record data of target association records including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively.

The predicted association degree determination unit is used for determining predicted degrees of association between the plurality of disease description entries and the plurality of genes respectively according to the determined predicted association scores.

Optionally, the predicted association degree determination unit may be used for, for any gene: the association scores corresponding to the disease description entry and the gene identifier corresponding to the gene in the plurality of preset information gene association databases are summed to obtain an association value of the disease description entry and the gene.

Optionally, the predicted association degree determination unit may be used for, for any gene: the association scores corresponding to the disease description entry and the gene identifier corresponding to the gene in the plurality of preset information gene association databases are weighted to obtain an association value of the disease description entry and the gene.

The predicted association degree determination unit may be used for determining the degree of association between the disease description entry and the gene according to the association value.

The error determination unit is used for determining errors between the predicted degrees of association and actual degrees of association of the plurality of disease description entries and the plurality of genes.

The actual degrees of association can be degrees of association between a plurality of disease description entries and a plurality of genes determined by professionals in the field.

The error comparison unit is used for determining whether the errors are below a preset error threshold, if yes, triggering the entry-gene association matrix determination unit, or if not, triggering the matrix parameter adjustment unit.

The entry-gene association matrix determination unit is used for determining the current entry-gene association training matrix as the entry-gene association matrix.

The matrix parameter adjustment unit is used for adjusting matrix parameters in the current entry-gene association training matrix through a gradient descent algorithm, using the adjusted entry-gene association training matrix as the current entry-gene association training matrix, and triggering the predicted association score determination unit 200.

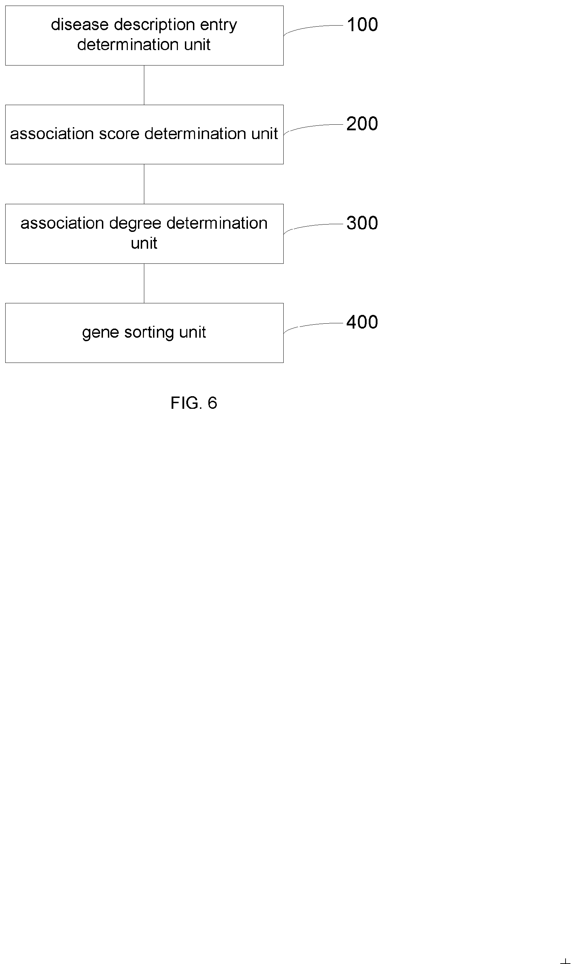

Optionally, based on the device shown in FIG.5, another device for determining a degree of gene association provided in some embodiments of the present application is shown in FIG. 6. The device may further comprise a gene sorting unit 400.

The gene sorting unit 400 may be used for sorting the plurality of genes in descending order of the degrees of association between the disease description entry and the plurality of genes after the association degree determination unit 300 determines the degrees of association between the disease description entry and the plurality of genes respectively.

The device for determining a degree of gene association comprises a processor and a memory. The disease description entry determination unit 100, the association score determination unit 200, and the association degree determination unit 300 are all stored in the memory as program units. The above program units are executed by the processor to implement corresponding functions.

The processor comprises a kernel, which calls the corresponding program units from the memory. One or more cores can be provided, through determining record data of target association records including a disease description entry and a plurality of genes in each preset association database, inputting the record data into a preset entry-gene association matrix, and determining an association score of the disease description entry with respective to each of the plurality of genes in the preset association database, and the degrees of association between the disease description entry and the plurality of genes can be rapidly obtained by adjusting core parameters.

Some embodiments of the present application provides a storage medium on which a program is stored, which when executed by a processor, implements the method for determining a degree of gene association.

Some embodiments of the present application provides a processor for executing a program, which when executed implements the method for determining a degree of gene association.

Some embodiments of this application provides an electronic device comprising at least one processor, at least one memory coupled to the processor, and a bus; wherein, the processor communicates with the memory through the bus; the processor is used for invoking program instructions in the memory to perform the above method for determining a degree of gene association. The electronic device herein may be a server, PC, PAD, or mobile phone, etc.

This application further provides a computer program product, which when executed on an electronic device, is suitable for executing a program that initializes the steps of the method for determining a degree of gene association.

The present application has been described with reference to flowcharts and/or block diagrams of methods, apparatuses, electronic devices (systems) and computer program products according to embodiments of the present application. It should be understood that each process and/or block in the flowcharts and/or block diagrams, and combinations of the processes and/or blocks in the flowcharts and/or block diagrams may be implemented by computer program instructions. The computer program instructions may be provided to a processor of a general purpose computer, a special purpose computer, an embedded processor, or other programmable device to generate a machine such that the instructions executed by a processor of a computer or other programmable data processing device to generate means implementing the functions specified in one or more flows of the flowcharts and/or one or more blocks of the block diagrams.

In a typical configuration, the electronic device includes one or more processors (CPU), a memory and a bus. The electronic device may further include an input-output interface, a network interface and the like.

The memory may include non-permanent memory in a computer-readable medium, random access memory (RAM) and/or non-volatile memory, such as read only memory (ROM) or flash RAM. The memory includes at least one memory chip. The memory is an example of a computer-readable medium.

Computer-readable media include permanent and non-permanent, removable and non-removable media, and information storage can be realized by any method or technology. The information can be computer-readable instructions, data structures, program modules, or other data. Examples of computer storage media include, but are not limited to, phase change memory (PRAM), static random access memory (SRAM), dynamic random access memory (DRAM), other types of random access memory (RAM), read-only memory (ROM), electrically erasable programmable read-only memory (EEPROM), flash memory or other memory technologies, CD-ROM, digital versatile disc (DVD) or other optical storage, magnetic cassettes, magnetic tape, magnetic disk storage or other magnetic storage devices, or any other non-transmission media that can be used to store information that can be accessed by computing devices. According to the definition herein, computer-readable media does not include transitory media, such as modulated data signals and carrier waves.

Further, terms "include", "comprise" or their any other variations are intended to encompass non-exclusive composition, so that a process, method, product or device comprising a series of factors may comprise not only these factors, but also other factors that are not listed explicitly, or factors intrinsic to this process, method, product or device. Without limitation, a factor defined by wording "comprise one..." does not exclude the existence of other same factors in a process, method, product or device comprising such factor.

One skilled in the art should understand that, the embodiments of the present application may be provided as a method, a system, or a computer program product. Therefore, embodiments of the present application can take the form of an entirely hardware embodiment, an entirely software embodiment or an embodiment containing both hardware and software elements. Moreover, the present application can be in a form of one or more computer program products containing the computer-executable codes which can be implemented in the computer-executable storage medium (including but not limited to disks, CD-ROM, optical disks, etc.).

The above are only examples of this application, and are not used to limit this application. For those skilled in the art, this application can have various modifications and changes. Any modifications, equivalent replacements, or improvements made within the spirit and principle of the present application shall be included in the scope of the claims of the present application.

## Claims

1. A method for determining a degree of gene association, **characterized by** comprising:

   determining a disease description entry in a case description text;
   for a plurality of preset association databases:

      determining record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes;
      inputting the record data into a preset entry-gene association matrix, to determine an association score of the disease description entry with respective to each of the plurality of genes in the preset association database; and

   determining degree of association between the disease description entry and each of the plurality of genes according to association scores of the disease description entry with respect to the gene identifiers corresponding to the plurality of genes in the plurality of preset association databases.

2. The method according to claim 1, **characterized in that** the determining record data of target association record(s) in the preset association database according to the disease description entry and gene identifiers each corresponding to one of a plurality of genes comprises:
   for any gene of the plurality of genes, determining record data of the target association record(s) of the disease description entry and a gene identifier corresponding to the gene in the preset association database respectively, wherein the record data of the target association record(s) comprises a first number of association record(s) including both the gene identifier of the gene and the disease description entry, a second number of association record(s) including the disease description entry, a third number of association record(s) including the gene identifier of the gene, and a total number of association record(s) in the preset association database.

3. The method according to claim 2, **characterized in that** the preset entry-gene association matrix is:

$$M = \frac{T_1}{T_2} \times \log\left(\frac{T_s}{T_3 + 1}\right)$$

wherein, M is the association score of the disease description entry with respect to the gene identifier corresponding to the gene in the preset information gene database; $T_1$ is the first number, $T_2$ is the second number, $T_3$ is the third number; $T_S$ is the total number.

4. The method according to claim 1, **characterized in that** the determining degree of association between the disease description entry and each of the plurality of genes according to association scores of the disease description entry with respect to the gene identifiers corresponding to the plurality of genes in the plurality of preset association databases comprises, for any gene:

   weighting the association scores of the disease description entry with respect to the gene identifier corresponding to the gene in the plurality of preset information gene association databases to obtain an association value between the disease description entry and the gene; and determining the degree of association between the disease description entry and the gene according to the association value.

5. The method according to claim 4, **characterized in that** the determining the degree of association between the disease description entry and the gene according to the association value comprises:

determining the degree of association between the disease description entry and the gene according to

$$L = \frac{1}{\left(1 + e^{-z}\right)}$$

, wherein L is the degree of association between the disease description entry and the gene, e is the natural constant, and z is the association value.

6. The method according to claim 1, **characterized in that** a training process of the preset entry-gene association matrix comprises:

inputting, as training information, record data of target association record(s) including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively;
determining predicted degrees of association between the plurality of disease description entries and the plurality of genes respectively according to the determined predicted association scores;
determining errors between the predicted degrees of association and actual degrees of association of the plurality of disease description entries and the plurality of genes;
determining whether the errors are below a preset error threshold;
if yes, determining that the current entry-gene association training matrix is the entry-gene association matrix; and
if not, adjusting matrix parameters in the current entry-gene association training matrix through a gradient descent algorithm, using the adjusted entry-gene association training matrix as the current entry-gene association training matrix, and turning to the step of inputting, as training information, record data of target association records including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively.

7. A device for determining a degree of gene association, **characterized by** comprising a disease description entry determination unit, an association score determination unit, and an association degree determination unit;

wherein the disease description entry determination unit is used for determining a disease description entry in a case description text;
the association score determination unit is used for, for a plurality of preset association databases: determining record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and inputting the record data into a preset entry-gene association matrix, to determine an association score of the disease description entry with respective to each of the plurality of genes in the preset association database; and
the association degree determination unit is used for determining degree of association between the disease description entry and each of the plurality of genes according to association scores of the disease description entry with respect to the gene identifiers corresponding to the plurality of genes in the plurality of preset association databases.

8. The device according to claim 7, **characterized in that** the association score determination unit is specifically used for, for any gene of the plurality of genes, respectively determining record data of target association record(s) including the disease description entry and a gene identifier corresponding to the gene in the preset association database, the record data of the target association record(s) comprising: a first number of association record(s) including both the gene identifier of the gene and the disease description entry, a second number of association record(s) including the disease description entry, a third number of association record(s) including the gene identifier of the gene, and a total number of association record(s) in the preset association database.

9. The method according to claim 8, **characterized in that** the preset entry-gene association matrix is:

$$M = \frac{T_1}{T_2} \times \log\left(\frac{T_s}{T_3 + 1}\right)$$

wherein, $M$ is the association score of the disease description entry with respect to the gene identifier corresponding to the gene in the preset information gene database; $T_1$ is the first number, $T_2$ is the second number, $T_3$ is the third number; $T_S$ is the total number.

10. The device according to claim 7, **characterized in that** the association degree determination unit comprises an association value obtaining subunit and an association degree determination subunit,

wherein the association value obtaining subunit is used for, for any gene, weighting the association scores of the disease description entry with respect to the gene identifier corresponding to the gene in the plurality of preset information gene association databases to obtain an association value between the disease description entry and the gene;
the association degree determination subunit is used for determining the degree of association between the disease description entry and the gene according to the association value.

11. The device according to claim 10, **characterized in that** the association degree determination subunit is used for determining the degree of association between the disease description entry and the gene

$$L = \frac{1}{\left(1 + e^{-z}\right)}$$

according to , wherein L is the degree of association between the disease description entry and the gene, e is the natural constant, and z is the association value.

12. The device according to claim 7, **characterized in** further comprising: a predicted association score determination unit, a predicted association degree determination unit, an error determination unit, an error comparison unit, an entry-gene association matrix determination unit, and a matrix parameter adjustment unit,

wherein the predicted association score determination unit is used for inputting, as training information, record data of target association records including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively;
the predicted association degree determination unit is used for determining predicted degrees of association between the plurality of disease description entries and the plurality of genes respectively according to the determined predicted association scores;
the error determination unit is used for determining errors between the predicted degrees of association and actual degrees of association of the plurality of disease description entries and the plurality of genes;
the error comparison unit is used for determining whether the errors are below a preset error threshold, if yes, triggering the entry-gene association matrix determination unit, or if not, triggering the matrix parameter adjustment unit;
the entry-gene association matrix determination unit is used for determining the current entry-gene association training matrix as the entry-gene association matrix;
the matrix parameter adjustment unit is used for adjusting matrix parameters in the current entry-gene association training matrix through a gradient descent algorithm, using the adjusted entry-gene association training matrix as the current entry-gene association training matrix, and triggering the predicted association score determination unit.

13. A storage medium, **characterized in that** a program is stored in the storage medium, which when executed by a processor implements the method for determining the degree of gene association according to any one of claims 1 to 6.

14. A processor, **characterized in that** the processor is used to execute a program, wherein the program when executed performs the method for determining the degree of gene association according to any one of claims 1 to 6.

15. An electronic device, **characterized in** comprising at least one processor, at least one memory coupled to the processor, and a bus;
wherein, the processor communicates with the memory through the bus, and the processor is used for invoking program instructions in the memory to perform the method for determining the degree of gene association according to any one of claims 1 to 6.

16. A computer program product, **characterized in that** when executed on an electronic device, the computer program product is adapted to execute a program for initializing the steps of the method for determining the degree of gene association according to any one of claims 1 to 6.

determine a disease description entry in a case description text    ⌐ S100

for each of a plurality of preset association databases, determine record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and input the record data into a preset entry-gene association matrix, to determine an association score of the disease description entry with respective to each of the plurality of genes in the preset association database    ⌐ S200

determine degree of association between the disease description entry and each of the plurality of genes according to association scores of the disease description entry with respect to the gene identifiers corresponding to the plurality of genes in the plurality of preset association databases    ⌐ S300

Fig. 1

determine a disease description entry in a case description text — S100

for a plurality of preset association databases, determine record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and input the record data into a preset entry-gene association matrix, to determine an association score of the disease description entry with respective to each of the plurality of genes in the preset association database — S200

for any gene, weight the association scores of the disease description entry with respect to the gene identifier corresponding to the gene in the plurality of preset information gene association databases to obtain an association value between the disease description entry and the gene — S310

determine the degree of association between the disease description entry and the gene according to the association value — S320

FIG. 2

input, as training information, record data of target association records including a plurality of disease description entries and gene identifiers of a plurality of genes in the plurality of preset information gene association databases into a current entry-gene association training matrix, and determining predicted association scores corresponding to the training information and the plurality of genes in the preset information gene association databases respectively — S10

determine predicted degrees of association between the plurality of disease description entries and the plurality of genes according to the determined predicted association scores — S20

determine errors between the predicted degrees of association and actual degrees of association of the plurality of disease description entries and the plurality of genes — S30

no

determine whether the errors are below a preset error threshold — S40

yes

determine that the current entry-gene association training matrix is the entry-gene association matrix — S50

adjusting matrix parameters in the current entry-gene association training matrix through a gradient descent algorithm, using the adjusted entry-gene association training matrix as the current entry-gene association training matrix — S60

FIG. 3

determine a disease description entry in a case description text ⎯ S100

for a plurality of preset association databases, determine record data of target association record(s) in the preset association database according to the disease description entry and gene identifiers each corresponding to one of a plurality of genes, wherein each of the preset association databases stores association records of the disease description entry and gene identifiers corresponding to at least one of the plurality of genes; and input the record data into a preset entry-gene association matrix, to determine an association score of the disease description entry with respective to each of the plurality of genes in the preset association database ⎯ S200

determine degree of association between the disease description entry and each of the plurality of genes according to association scores of the disease description entry with respect to the gene identifiers corresponding to the plurality of genes in the plurality of preset association databases ⎯ S300

sort the plurality of genes in descending order of the degrees of association between the disease description entry and the plurality of genes ⎯ S400

FIG. 4

disease description entry determination unit ⎯ 100

the association score determination unit ⎯ 200

the association degree determination unit ⎯

FIG. 5

FIG. 6

## INTERNATIONAL SEARCH REPORT

| International application No. |
|---|
| **PCT/CN2021/073013** |

| A. | CLASSIFICATION OF SUBJECT MATTER |
|---|---|

G06F 16/33(2019.01)i; G06F 40/295(2020.01)i; G06K 9/62(2006.01)i; G16H 50/70(2018.01)i; G16B 50/00(2019.01)i

According to International Patent Classification (IPC) or to both national classification and IPC

| B. | FIELDS SEARCHED |
|---|---|

Minimum documentation searched (classification system followed by classification symbols)

G06F; G06K; G16H; G16B

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 基因, 关联, 疾病, 描述, 病历, 数据库, 数量, 数目, 分数, 分值, 加权, 权值, genes, association, diseases, description, medical w record, database, quantity, number, score, weight

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 109119132 A (RESEARCH INSTITUTE OF PRC NATIONAL HEALTH AND FAMILY PLANNING COMMISSION) 01 January 2019 (2019-01-01)<br>description paragraphs 0071-0078 | 1-16 |
| Y | CN 110349632 A (SEQUMED BIOTECHNOLOGY INC.) 18 October 2019 (2019-10-18)<br>description, paragraphs 0020-0031 | 1-16 |
| A | US 2019035506 A1 (HEFEI UNIVERSITY OF TECHNOLOGY) 31 January 2019 (2019-01-31)<br>entire document | 1-16 |
| A | CN 102855398 A (INSTITUTE OF AUTOMATION, CHINESE ACADEMY OF SCIENCES) 02 January 2013 (2013-01-02)<br>entire document | 1-16 |
| A | CN 110534159 A (CHINESE PLA GENERAL HOSPITAL) 03 December 2019 (2019-12-03)<br>entire document | 1-16 |

☐ Further documents are listed in the continuation of Box C. ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 September 2021** | **24 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/073013**

| Patent document cited in search report | | | Publication date (day/month/year) | Patent family member(s) | | | Publication date (day/month/year) |
|---|---|---|---|---|---|---|---|
| CN | 109119132 | A | 01 January 2019 | HK | 40000913 | A1 | 23 October 2020 |
| CN | 110349632 | A | 18 October 2019 | None | | | |
| US | 2019035506 | A1 | 31 January 2019 | CN | 107403068 | A | 28 November 2017 |
| CN | 102855398 | A | 02 January 2013 | None | | | |
| CN | 110534159 | A | 03 December 2019 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202011535972